# EUROPEAN PATENT SPECIFICATION

(11) **EP 0 556 564 B1**
(45) Date of publication and mention of the grant of the patent: **06.08.1997**
(21) Application number: 93100577.1
(22) Date of filing: 15.01.1993
(51) Int. Cl.: A61B 17/00, A61B 17/12

(54) **System for the percutaneous transluminal delivery and retrieval of a prosthetic occluder**
System zum perkutanen, transluminalen Anbringen und Herausnehmen einer Verschlussprothese
Système pour l'application et l'extraction percutanée, transluminale d'un élément prothétique d'occlusion

(30) Priority: 22.01.1992 US 824019
(43) Date of publication of application: 25.08.1993
(73) Proprietor: C.R. BARD, INC., Murray Hill New Jersey 07974 (US)
(72) Inventor: Bourne, George, Groton, MA 01450 (US); Duval, George, Lake George, N.Y. 12845 (US); Goodine, Dennis, Dracut, MA 01826 (US); Ouellette, Gerry, Framingham, MA 01701 (US); Whittaker, Gregory R., Burlington, MA 01803 (US)
(74) Representative: Gossel, Hans K., Dipl.-Ing.

(56) References cited:
- EP-A- 0 113 520
- EP-A- 0 359 447
- EP-A- 0 397 038
- DE-A- 3 818 279
- US-A- 3 874 388
- US-A- 4 865 017
- CIRCULATION vol. 75, no. 3, March 1987, pages 583 - 592 RASHKIND ET AL. 'Non surgical closure of patent ductus arteriosus'

## Description

### FIELD OF INVENTION

The present invention relates to a front-end loading device for transluminal delivery of a collapsible prosthetic occluder and a method of delivering a prosthetic device into the lumen of a percutaneous transluminal introducer sheath utilizing such a device.

### BACKGROUND OF THE INVENTION

Various prosthetic occluders for repairing intracardiac defect, such as interarterial and interventricular septal shunts, patent ductus arteriosus and aortico-pulmonary window have been proposed by the prior art. Representative is U.S. Patent No. 3,874,388 to King et al. which discloses a device including a pair of opposed umbrella-like occluder elements which lock together at a central hub extending across the defect.

The King patent describes an assembly for percutaneous transluminal delivery of the umbrella-like occluder including a delivery cone, a catheter and an obturator guidewire. The distal end of the delivery cone is inserted into the proximal end of the catheter and includes a cone-shaped lumen which compresses the occluder as it is advanced from the delivery cone to the catheter lumen in a procedure known as "front-end loading". The obturator guidewire extends through the delivery cone and the catheter lumen and is threaded at a distal tip to the occluder. Manipulation of the proximal end (outside of the patient) of the obturator wire reciprocally advances the collapsed umbrella-like occluder transluminally to the septal or ductus defect. Unscrewing the distal tip of the obturator wire, by rotating the proximal end, releases the deployed umbrella-like occluder from the delivery system.

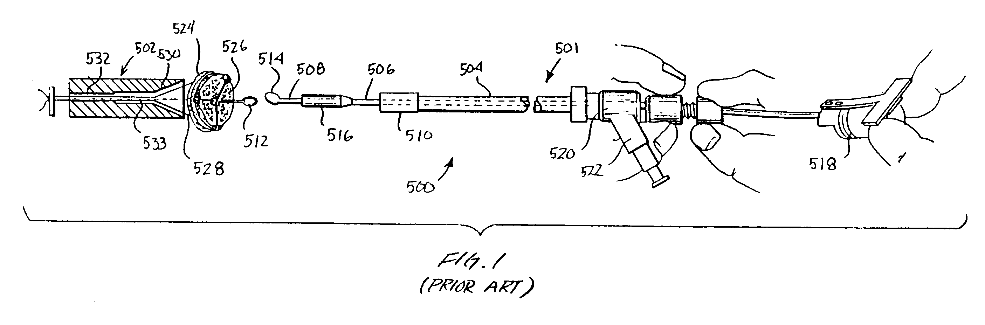

A prosthetic occluder delivery system is described in W.J. Rashkind et al., "Non Surgical Closure of Patent Ductus Arteriosus: Clinical Application of the Rashkind PDA Occluder System," Circulation, Vol. 75, No. 3, March 1987, pg. 583-592. The Rashkind rear-end loading delivery system 500 illustrated in Fig. 1 includes a delivery assembly 501 and a separate loading device 502 for collapsing and then rear-end loading the collapsed occluder 524, 526 into the delivery assembly 501. The delivery assembly 501 includes an 85 cm long 8 French catheter 504, a delivery wire 506 and a locking wire 508. The distal tip of the catheter 504 includes a metal tubular pod 510 having a central lumen sized to maintain the occluder in the collapsed configuration. The delivery wire 506 and the locking wire 508 extend through the catheter 504 and beyond the pod 510. An attachment eye 512 extending from the occluder is seatable about a hemispherical-shaped knuckle 514 at the distal end of the locking wire 508. The lumen of a metal sleeve 516 at the distal end of the delivery wire 506 is sized to prevent the detaching of the knuckle 514 and attachment eye 512. The delivery wire 506 and locking wire 508 are axially moveable relative to one another and to the metal sleeve 516 by manipulation of a piston-cylinder control handle 518. A back bleed gasket assembly 520 at the proximal end of the catheter includes a side leg 522 for infusing liquid along the entire length of the catheter 504 and through the pod 510 to aspirate air bubbles from the compressed occluder.

The Rashkind procedure for loading the occluder, known as "rear-end loading", begins with the connection of the proximal occluder element 524 to the delivery assembly 501. The knuckle 514 is advanced distally of the metal sleeve 516 by closing the control handle 518. The attachment eye 512 is placed around the knuckle 514 so that the occluder elements 524, 526 are perpendicular to the longitudinal axis of the catheter 504. Extension of the control handle 518 draws the seated attachment eye 512 and knuckle 514 into the metal sleeve 516.

Tension applied to the sutures 528 extending through the loading device 502 causes the distal occluder element 524 to fold into the conical portion 530. As the sutures 528 are pulled through the loading device 502, the following proximal occluder element 526 folds backwards and is drawn into the conical portion 530. The occluder is pulled through the loading device 502 until it completely collapses in the distal portion 532. The pod 510 is advanced over the delivery wire 506 into the middle section 533 of the loading device 502. After cutting the sutures, the delivery wire is retracted which in turn draws the collapsed occluder completely into the pod 510. After removing the pod 510 from the loading device 502, the rear-end loaded collapsed occluder is flushed by infusing liquid from the side leg 522 all the way down the catheter 504 and through the pod 510. The flushed delivery assembly 501 is inserted into an already emplaced introducer sheath and then the pod 510 is transluminally advanced towards the defect.

The Rashkind rear-end loading system suffers from several deficiencies. It requires coordination of a separate loading cone and delivery assembly to collapse the occluder and then deliver the collapsed occluder into the introducer sheath. Handling of the lengthy and thick delivery catheter is awkward.

Aspiration and flushing of the occluder has proved inefficient because the infusion fluid must be injected 85 cm downstream of the collapsed occluder. Mating of the attachment eye and knuckle, which is required to connect the occluder to the delivery system, requires a practiced skill and may take several attempts even for the experienced physician. Further, the rigidity of the metal pod precludes the delivery system from being navigated along the twisted curvature of the blood vessels adjacent the defect site and therefore prevents the delivery system from transporting the occluder to the defect site.

From U.S. Patent No. 3,874,388 there is known a front-end loading delivery device according to the preamble of claim 1.

This document does not, however, refer to the provision of an infusion port to allow for the introduction of a flushing liquid.

### SUMMARY OF THE INVENTION

The present invention is a front-end loading device for transluminal delivery of a collapsible prosthetic occluder, comprising a main body having a distal end, a proximal end and a lumen extending therethrough, a portion of said lumen being tapered inwardly toward a smaller diameter distal portion, and a collapsible prosthetic device having a first configuration larger than said distal portion of said lumen and a second configuration smaller than said distal portion of said lumen, said collapsible prosthetic device being moveable in said second configuration through said distal portion of said lumen, wherein it further comprises an infusion port extending through said main body and in direct communication with said distal portion of said lumen.

The present invention further relates to a method of delivering a prosthetic device into the lumen of a percutaneous transluminal introducer sheath comprising collapsing the prosthetic device in the lumen of a delivery device, the delivery device including the features of the device according to claim 1; infusing fluid through a port extending into the delivery device and in communication with the delivery device lumen to remove air bubbles from the collapsed prosthetic device; and advancing the prosthetic device from the delivery device into the introducer lumen.

The present invention is usable in connection with a system for percutaneous transluminal delivery and retrieval of a prosthetic occluder used to repair congenital or acquired defects (shunts) in the heart or the major blood vessels thereof including interarterial and interventricular septal shunts, patent ductus arteriosus and aortico-pulmonary window. The percutaneous transluminal prosthetic occluder delivery and retrieval system includes a front-end loading delivery device, a control assembly and an introducer and retrieval sheath assembly.

The front-end loading delivery device includes a conical-shaped lumen for collapsing the prosthetic occluder into a narrow or slender configuration which is advanceable through the lumen of the introducer sheath. A side leg having an infusion port provides direct access into the lumen to aspirate air bubbles from the compressed occluder and a valve assembly seals the front-end loading delivery device from backflow of fluid. An elongated distal end of the front-end loading delivery device is inserted into a hub at the proximal end of the previously placed indwelling introducer sheath. The introducer sheath includes a flexible distal end which is bendable into a shape conforming to the curvature of the blood vessels adjacent the defect.

A control assembly guides the collapsed occluder from the front-end loading delivery device through the introducer sheath lumen and to the defect site. The control assembly includes a locking wire with a ball-head distal tip that is engageable with a ball-head tip of an extension arm of the collapsed occluder. An elongated tubular shaft surrounds the locking wire and includes a distal metal sleeve with an internally dimensioned lumen for holding the ball-head tips together, preventing disengagement of the control assembly and the occluder. Advancing the locking wire ball-head tip out of the metal sleeve releases a deployed occluder.

A retrieval sheath co-axially mounted about the introducer sheath includes a distal end which is less flexible than the distal end of the introducer sheath. A deployed but unreleased occluder may be retrieved by retracting the introducer sheath and control assembly against the distal end of the less flexible retrieval sheath until the expanded occluder everts into a narrow configuration withdrawable through the retrieval sheath lumen.

It is among the general objects of the invention to provide a front-end loading delivery device for inserting a collapsible occluder into an introducer sheath.

It is a further object of the invention to provide a front-end loading delivery tool for enhancing removal of air bubbles from a collapsed occluder element.

Other objects and features of the present invention will become apparent from the following detailed description when taken in connection with the accompanying drawings which disclose multiple embodiments of the invention. It is to be understood that the drawings are designed for the purpose of illustration only and are not intended as a definition of the limits of the invention.

### DESCRIPTION OF THE DRAWINGS

The foregoing and other objects and advantages of the invention will be appreciated more fully from the following drawings in which:
Fig. 1 is a fragmented illustration of the prior art Rashkind rear-end loading delivery system;
Fig. 2 is a fragmented illustration of the control assembly, front-end loading delivery device and introducer and retrieval sheath assembly in accordance with the invention;
Fig. 3 is a sectional illustration of the front-end loading delivery device in accordance with the invention;
Fig. 4 is an illustration, partly in phantom, of the cap of the front-end loading delivery device illustrated in Fig. 3;
Fig. 5 is an illustration, partly in section, of the compressible gland of the front-end loading delivery device shown in Fig. 3;
Fig. 6 is a fragmented illustration of the control assembly in accordance with the invention;
Fig. 7 is a fragmented illustration of the introducer and retrieval sheath assembly in accordance with the invention;
Figs. 8(a)-8(d) are schematic representations of a method of loading and delivering an occlusion device to repair a patent ductus arteriosus; and
Fig. 9 is a schematic representation of a method of retrieving a deployed occlusion device in accordance with the invention.

### DESCRIPTION OF THE PREFERRED EMBODIMENTS

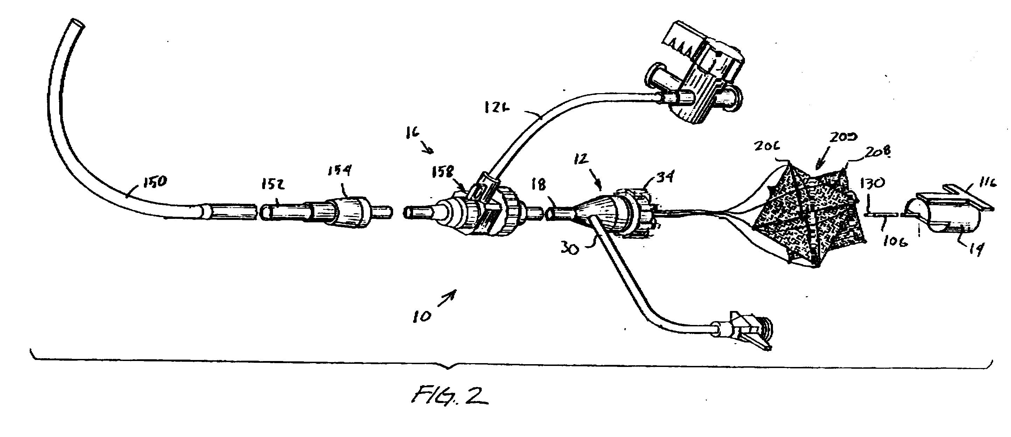

The system for percutaneous transluminal delivery and retrieval of a prosthetic occluder 10 shown in Fig. 2 includes a front-end loading delivery device 12, a control assembly 14, and an introducer and retrieval sheath assembly 16. The front-end loading delivery device 12 compresses the occluder into a narrow configuration which is suitable for loading into the lumen of the introducer and retrieval sheath assembly 16. After the occluder is collapsed, the distal end 18 of the front-end loading delivery device 12 is inserted into a hub at the proximal end of the previously placed indwelling introducer sheath. The proximal end of the control assembly 14 (outside of the patient) is manipulated to advance the occluder from the delivery device 12 into and through the introducer and retrieval sheath assembly 16 until it reaches the defect site where it is deployed in an open configuration. Activation of the control assembly 14 then releases the deployed, fully expanded occluder.

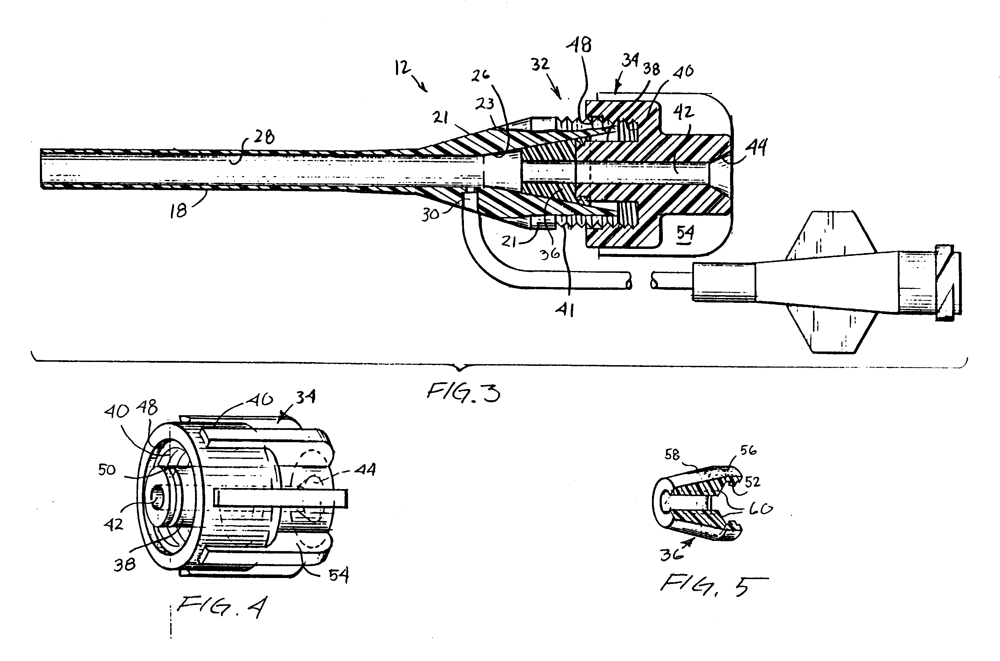

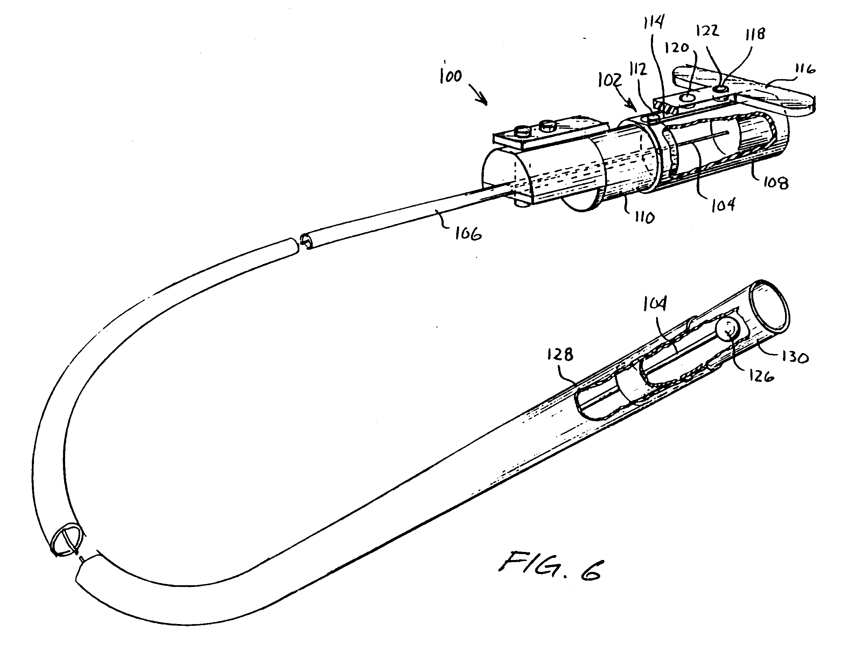

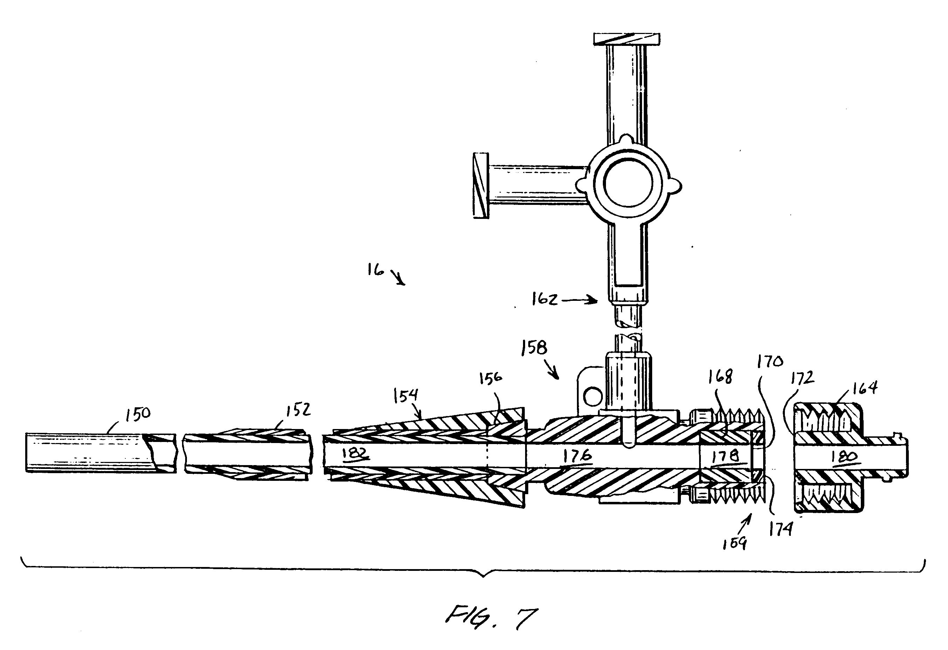

The front-end loading delivery device 12 illustrated in Figs. 3-5 includes a main body portion 21 having a tapering lumen portion 26, which preferably is conically shaped, and a uniform smaller diameter distal lumen portion 28 which is sized to compress the occluder to a predetermined diameter compatible with the introducer sheath lumen. A port 30 opens into the smaller diameter distal lumen portion 28 immediately adjacent the tapering distal portion 26. The port 30 is adapted to receive an infusion side leg or other structure for flushing saline or other fluids directly into the collapsed occluder to aspirate air bubbles therefrom. Preferably, the port 30 is positioned perpendicular to the longitudinal axis of the smaller diameter distal lumen portion 28 so that the infusion fluid is injected along a path perpendicular to the axis of the collapsed occluder.

A valve assembly 32 for sealing the lumen of the front-end loading delivery device 12 against backflow of the infusion fluid or blood is mounted to the proximal end of the main body portion 21. The valve assembly 32 includes a threaded cap 34 and a compressible gland 36. A shoulder or axial projection 38 compresses the gland 36 when the threaded annular rim 40 of the cap 34 is tightened to the threaded portion 41 of the main tubular body 21. The cap 24 includes a locking step projection 48 which prevents the cap from separating from the main body portion 21 when the cap is loosened. The unscrewed cap can only be removed by pulling the cap until the locking step 48 resiliently snaps under the depending neck wall 49 at the proximal end of the main body 21. A ribbed surface portion 54 of the cap promotes gripping and rotating of the cap by the physician. The cap includes a central lumen 42 which is sized for slidable passage of the elongated shaft of the control assembly 14. A tapering proximal portion 44 of the cap lumen 42 facilitates insertion of the elongated shaft.

The compressible gland 36 includes a cylindrical portion 56 and a conical portion 58. The cylindrical portion 56 is attached to the cap 34 and includes an annular projection 52 which mates with an annular groove 50 in the cap shoulder 38. Alternatively, the annular projection may extend from the cap shoulder into an annular groove in the gland. The proximal wall 60 of the conical portion 58 resiliently seats against the cap shoulder 38 and is the thrust bearing surface as the cap 34 is tightened. The shape of the conical portion 58, in the uncompressed state, closely matches the shape of the proximal end of the tapering lumen 26. The compressible gland 36 includes a central lumen 39 which in the non-compressed state has a diameter larger than the perimeter of the elongated shaft but smaller than the perimeter of the fully collapsed occluder. The assembled cap 34 and gland 36 define a continuous lumen 62 therethrough.

The cap and gland assembly is inserted into the proximal end of the main body portion 21 until the locking step resiliently snaps under the depending wall 49. Prior to tightening the cap 34, the conical portion 58 of the gland fills the proximal end of the tapering lumen 26. As the cap 34 is threaded to the main body portion 21, the gland 34 perimeter increasingly fills the unoccupied distal end of the tapering lumen 26 while the diameter of the gland lumen becomes increasingly smaller.

Preferably, the cap 34 and the tubular body 23 are formed of a rigid polycarbonate material which also is transparent so that the physician may observe the integrity of the prosthetic device during the loading procedure. Alternatively, the tubular body may be formed by insert molding the proximal end of a high density polyethylene tube having a uniform lumen diameter to a polycarbonate hub containing a tapering lumen. The gland 36 preferably is formed of a low durometer material such as a silicone or a latex.

In a representative embodiment, an 8 French front-end loading delivery device has a main body which is 2.345 inches (59.6 mm) long with the distal end being 1.522 inches (38.7 mm) long and the proximal section being 0.823 inches (20.9 mm) long. The tapering portion of the lumen has an inner diameter decreasing from 0.320 inches (8.13 mm) to 0.128 inches (3.25 mm) while the distal portion of the lumen has a uniform diameter of 0.128 inches (3.25 mm). The distal end of the main body portion has an outside diameter of 0.122 inches (3.10mm). The infusion port is spaced slightly from the distal end of the tapering portion of the lumen and has an inner diameter of .07 inches (1.78 mm) that steps down to an inner diameter of 0.04 inches (1.02 mm) at the juncture with the distal lumen portion. The cap is 0.637 inches (16.18 mm) long with a through-lumen diameter of 0.09 inches (2.29 mm). The compressible gland is 0.35 inches (8.89 mm) long with a lumen diameter in the non-compressed state of 0.08 inches (2.03 mm).

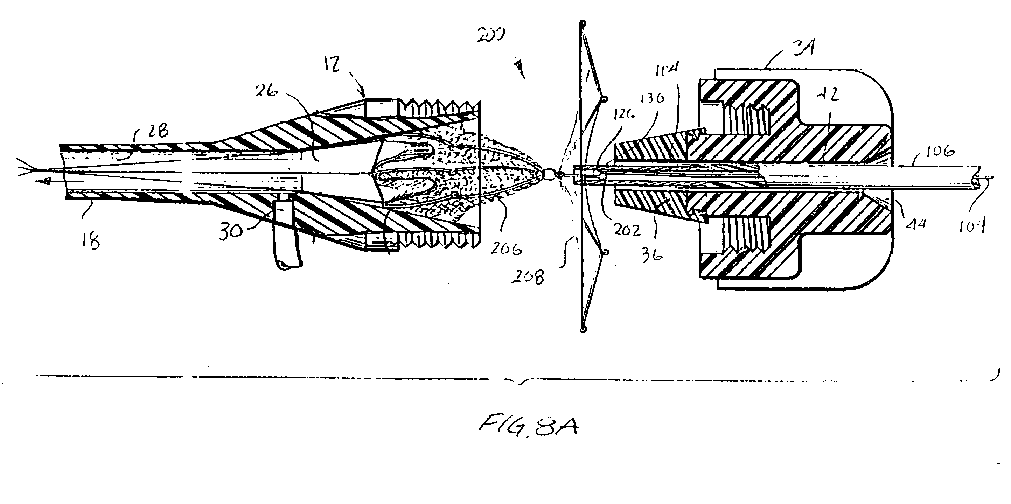

The control assembly 100 includes a plastic handle 102, a locking wire 104 and an elongated tubular shaft 106 as illustrated in Fig. 6. The handle 102 controls relative movement of the locking wire 104 and the elongated shaft 106 and includes a telescopically arranged cylinder 108 and piston 110 formed of a polycarbonate material. The proximal end of the locking wire 104 preferably is fixed to the cylinder 108 by set screws or other means apparent to one of skill in the art while the proximal end of the elongated shaft 106 may be bonded to the piston 110 with an adhesive such as FDA-2 epoxy. The distal end of the locking wire includes a locking member that preferably is a spherical or ball-shaped enlargement 126 of the distal tip of the locking wire 104. The ball-head 126 may be formed by stamping or pressing the distal tip of the locking wire 104. Alternatively, a separate ball-shaped member may be soldered, welded or brazed to the distal tip of the locking wire as would be apparent to those of skill in the art.

The elongated shaft 106 includes a hypodermic tube 107 and a non-thrombogenic plastic jacket 109 which encapsulates the hypodermic tube 107. The hypodermic tube 107 preferably is formed of type 304 stainless steel and the non-thrombogenic jacket preferably is formed of a polyurethane such as Pebax 7033 distributed by Atochem. The distal end of the jacket 109 extends beyond the distal tip of the hypodermic tube 107 to form a flexible distal segment 128 of the elongated shaft 106. The flexible distal segment 128 contains a less flexible member such as a small metal sleeve 130 which prevents the ball-head tip 126 and the ball-head end of the occluder extension arm from detaching as discussed below. When the ball-head tip 126 and the ball-head end have the same spherical shape and size, the small metal sleeve 130 has an inner diameter that ranges between the diameter of the spherical enlargement 126 and twice the diameter of the spherical enlargement 126 and, preferably, is slightly smaller than twice the diameter of the spherical enlargement 126.

The telescoping arrangement of the cylinder 108 and piston 110 controls the relative position of the locking member and the metal sleeve. In the fully closed position of the handle 102, the spherical enlargement 126 extends beyond the small metal sleeve 130. In the retracted position, the spherical enlargement 126 is positioned within the small metal sleeve 130. A stop 112 extending from the piston 110 and an elongated groove 114 on the cylinder 108 cooperate to limit the extension of the handle 102. Respective apertures 120, 122 on a T-shaped plastic tab 116 are selectively engageable with a locking member 118 to secure the handle 102 in a retracted or a closed position.

In a representative embodiment, the hypodermic tube has a length of 45.01 inches (1143 mm) with an inner diameter of 0.034 inches (0.86 mm) and an outer diameter of 0.048 inches (1.22 mm). The non-thrombogenic plastic jacket portion encapsulating the hypodermic tube has an outer diameter of 0.064 inches (1.63 mm) while the flexible distal end has an outer diameter of 0.056 inches (1.42 mm), an inner diameter of 0.048 (1.22 mm) and a length of 5.9 inches (149.9 mm). The hypodermic tube and non-thrombogenic plastic jacket are joined by sliding an extruded plastic jacket over an FDA-2 epoxy adhesive coated hypodermic tube. The locking wire is formed of type 304 stainless steel and has a length of 45.6 inches (1158 mm) and a diameter of 0.014 inches (0.36 mm). The enlarged distal tip of the locking wire has a diameter of 0.025 inches (0.64 mm). The distal metal sleeve has a lumen diameter of 0.040 inches (1.02 mm) and an outside diameter of 0.056 inches (1.42 mm). A proximal extension of the distal metal sleeve has an outer diameter of 0.040 inches (1.02 mm) and a length of 0.025 (0.64 mm) inches. The proximal extension is coated with FDA-2 epoxy adhesive and inserted into the distal end of the non-thrombogenic plastic jacket to join the metal sleeve to the elongated shaft.

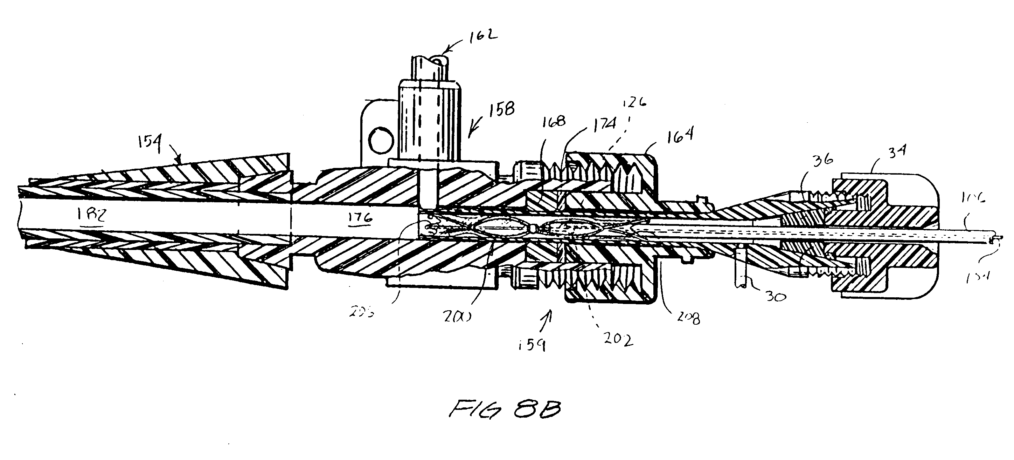

The introducer and retrieval sheath assembly 16 illustrated in Fig. 7 provides the transluminal pathway to the defect site for the occluder and includes a long flexible introducer sheath 150 and a shorter rigid retrieval sheath 152. The distal end of the introducer sheath is bendable into a shape that conforms with the vasculature adjacent the defect; for example, the curve of the pulmonary artery and the ductus when repairing a patent ductus arteriosus. The retrieval sheath 152 is less flexible than the distal portion of the introducer sheath 150 and is not similarly bendable. The introducer sheath 150 preferably is a single lumen tube with an inner lining of Teflon and an outer jacket formed of a polyurethane material such as Pebax 6333 distributed by Ato Chemical. The introducer sheath 150 preferably is formed by supporting a Teflon cylindrical liner on a mandrel and heat shrinking a polyurethane jacket thereover. A layer of FDA-2 epoxy adhesive may be interposed between the liner and the jacket to further secure the elements together. A process for surface treatment of the Teflon may be required to facilitate the bonding to the outer jacket. This may include molecular restructuring using plasma etching technologies or chemical treatment using etchants such as Tetra-Etch distributed by W.L. Gore & Associates, Inc. The mandrel preferably is curved at a distal tip to heat set the introducer sheath with a propensity for forming a curve similar to the curve of the body lumen adjacent the defect site. The retrieval sheath may be formed of a rigid polyurethane such as Isoplast 101 distributed by Dow Chemical.

A hub and flange assembly 151 releasably connects the introducer and retrieval sheaths 150, 152. The polymeric cone-shaped flange 154 may be made of Kraton distributed by Shell Chemical Co. and preferably is bonded with FDA-2 epoxy adhesive at a distal end to the retrieval sheath 152 and is releasably connected at a flexible proximal end to a less flexible tapering projection 156 of the hub 158. The cone-shaped flange 154 includes a lumen with a constant inner diameter distal portion and a flexible tapering proximal portion that is adapted to receive the less flexible tapering projection 156 in a tight fitting male/female relationship. The distal end of the hub 158 is insert molded to the proximal end of the introducer sheath 150.

A side leg 162 extending from the hub 158 is adapted to receive a syringe for flushing the introducer sheath. A valve 159 is connected to a proximal end of the hub 158 and includes a cap 164 and a compressible cylindrical-shaped gland 168 which seats within a valve chamber 170 within the hub 158. An axially projecting annular shoulder 172 compresses the gland 168 when the cap 164 is tightened to the hub 158. A Teflon washer 174 preferably is disposed between the proximal end of the gland 168 and the annular shoulder 172 and prevents rotation of the gland as the cap 164 tightens to the hub 158. The hub lumen 176, the gland lumen 178 and the cap lumen 180 form a continuous passageway through the hub into the proximal end of the introducer sheath lumen and is sized to receive the distal end of the front-end loading delivery device.

In a representative embodiment an 8 French introducer sheath has an inner diameter of 0.108 inches (2.74 mm), an outer diameter of 0.130 inches and a length of 33.46 inches (3.30 mm). The retrieval sheath has an inner diameter of 0.158 inches (4.01 mm), an outer diameter of 0.178 inches (4.52 mm) and a length of 7.87 inches (200 mm). The cone-shaped flange is 1.120 inches (28.4 mm) long with an outside diameter that tapers from 0.415 inches (1.05 mm) to 0.240 inches (6.10 mm). The tapering portion of the flange lumen ranges from 0.400 inches (1.02 mm) to 0.180 inches (4.57 mm) and has a length of 0.317 inches (8.05 mm).

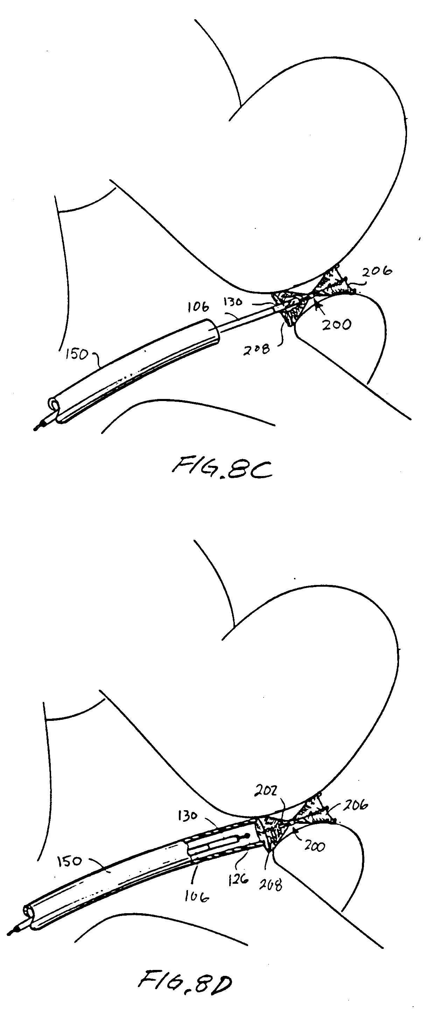

The loading and delivery of a prosthetic device is shown in Figs. 8(a)-8(d). While the operation of the invention is discussed in connection with the repair of a patent ductus arteriosus, a similar loading and delivery procedure would be followed for the occlusion of arterial and septal defects. The prosthetic device 200 is a collapsible occluder, such as the occluder disclosed in the commonly assigned application for "Occluder and Method of Repair of Cardiac and Vascular Defects", filed in the name of Dr. James E. Lock, George Duvall and Rudy Davis, on November 5, 1991. A typical occluder has diametrically opposed umbrella-like elements which are connected in a face-to-face relationship by a central hub. The occluder framework can be collapsed and then automatically opened by resilient means which are provided in the elongated struts. A patch material is held in place by the strut framework and serves to cover and thereby occlude the shunt defect. At least one strut of the occluder element is provided with a radiopaque material to allow fluoroscopic visualization of the occluder during the catheterization procedure. Extending from the central hub through one of the occluder elements is an elongated locking arm which includes a ball-head 202 at its distal tip. The ball-head 202 preferably is approximately the same size and shape as the spherical enlargement 126 of the locking wire 104. Suture lines 204 connect the peripheral struts of the distal occluder element in parachute-like fashion so that the occluder can be drawn into the conical portion of the front-end loading delivery device 12.

Prior to insertion, the occluder 200 is soaked in normal saline. A syringe is used to flush further saline through the patch material to force air bubbles out through the occluder surface. The cap 34 and compressible gland 36 are threaded over the elongated shaft so that they are positioned proximally of the locking sleeve 130. The occluder 200 is secured to the control assembly 14 by inserting the ball-head end 202 of the extension arm into the metal sleeve 130 and retracting the piston-end of the handle so that the enlarged spherical projection 126 reciprocally moves into the sleeve 130 in abutting contact with the sleeve and the ball-head 202. The internal perimeter of the sleeve 130 prevents either of the ball-heads from escaping around the other. The T-shaped plastic tab is locked to the handle ensuring that the occluder is securely retained to the control assembly.

With one person holding the control assembly, another person draws the suture lines 204 through the lumen of the front-end loading delivery device 12 which in turn causes the distal occluder element 206 to collapse inwardly. The folded distal occluder element 200 is advanced through the conical lumen portion 26 and then is pulled into and through the smaller diameter lumen 28 portion where it is tightly compressed. The proximal occluder element 208 is reciprocally advanced through the tapering lumen portion 26 until its legs fold rearwardly. The suture lines 204 are pulled through the distal end of the delivery device 12 until both occluder elements 206, 208 are tightly compressed within the uniformed diameter distal portion 28 of the lumen. The sutures 204 are cut and removed from the distal occluder element 206 and then the cap 34 is tightened to the main body of the delivery device so that the compressible gland 36 seals the tapering portion 26 of the delivery device lumen about the elongated shaft. A syringe is attached to the side leg 30 and fluid, such as saline, is infused into the smaller diameter lumen 26 and into and across the collapsed prosthetic device to force any entrained air bubbles to exit through the distal portion of the lumen. Once sufficiently aspirated, the assembly is ready for insertion into the introducer sheath.

The introducer and retrieval sheath assembly previously has been placed in the patient following a standard right heart catheterization. Briefly, that procedure involves cannulating the right femoral vein with an 8 French introducer sheath and then manipulating a 7 French end hole angiocatheter through the right heart, the pulmonary artery, the ductus and into the descending aorta just distal to the ductus. An angiocardiogram may be performed to determine the ductus shape, size and anatomy. After the angiocardiogram, the angiocatheter is replaced with a 7F or 8F end hole catheter which is readvanced through the ductus and into the descending aorta. An exchange guidewire is passed through the catheter into the descending aorta. With a guidewire placed in the ductus, the end hole catheter is replaced with the introducer and retrieval sheath assembly which is advanced over the guidewire through the right heart, the pulmonary artery and ductus so that the tip of the assembly lies at the aortic end of the ductus. To facilitate steering and manipulation of the assembly, the flexible distal end of the introducer sheath may be pre-bent to conform to the curve of the pulmonary artery and ductus. The exchange guidewire, and a dilator if used to predilate the vascular route, are removed and the introducer and retrieval assembly is flushed to eliminate air and any clots.

The distal end of the front-end loading device 12 is inserted into the hub 158 which is attached to the proximal end of the introducer sheath 150 as illustrated in Fig. 8(b). The cap 164 is tightened to secure the delivery device 12 to the introducer and retrieval sheath assembly 16. Forward movement of the control assembly (outside of the patient) advances the collapsed occluder 200 from the distal lumen portion of the delivery device 12 into the introducer sheath lumen. At this time, the introducer sheath 150 may be flushed by infusing an appropriate solution through the side leg 162.

The control assembly is advanced slowly and carefully under fluoroscopy until the legs of the distal occluder element 206 spring open in the descending aorta as illustrated in Fig. 8(c). The control assembly and introducer sheath 150 are retracted together towards the ductus until the flexing of the distal legs and the conical end of the ductus is observed. While holding the control assembly, the introducer sheath 150 is withdrawn which allows the struts of the proximal occluder element 208 to spring open on the pulmonary side of the ductus. The introducer sheath 150 is readvanced against the proximal occluder element 208 to position the occluder 200. Closing the control handle advances the spherical enlargement 126 beyond the metal sleeve 130 and releases the ball-head locking member 202 of the deployed occluder 200 from the control assembly 14 as shown in Fig. 8(d). The control assembly may then be removed through the introducer sheath 150.

An angiocardiogram in the descending aorta adjacent to the ductus is performed ten to fifteen minutes after deployment to provide sufficient time for ingrowth of thrombus into the occluder. After determining that the placement and operation of the occluder is acceptable, the introducer and retrieval sheath assembly may be removed.

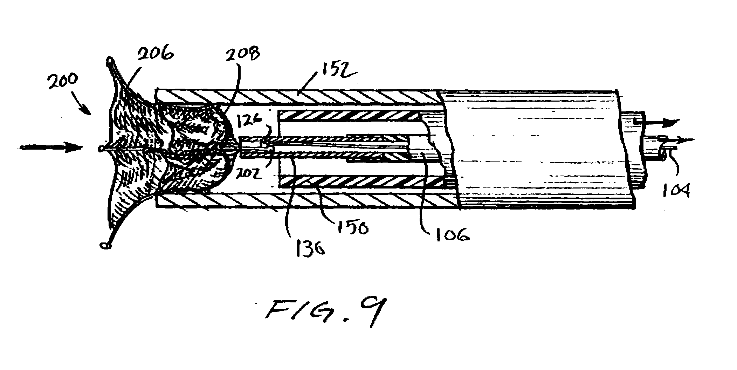

In certain circumstances retrieval of an expanded, but not as yet released, occluder is required, such as when fluoroscopy reveals that the occluder is damaged or too small to seal the ductus defect. The flexible distal portion of the introducer sheath, which is bendable into a shape that approximates the pulmonary artery curve, is not rigid enough to provide a thrust bearing surface against which to evert the resiliently expanded occluder. Removal of the occluder is accomplished by withdrawing the introducer sheath 150 and the control assembly, which is still connected to the occluder, through the retrieval sheath until the expanded proximal occluder element 208 contacts the rigid distal tip of the retrieval sheath 152 as illustrated in Fig. 9. The repeated retraction of the introducer sheath 150 and the control assembly eventually everts the proximal occluder element 208. The collapsed proximal occluder element 208 then may be withdrawn into the retrieval sheath lumen. The distal occluder element 206, which naturally folds in the direction of the retrieval sheath, easily collapses into the retrieval sheath lumen as the control assembly and introducer sheath are further withdrawn.

The present invention therefore provides a front-end loading device for transluminal delivery of collapsible prosthetic occluder and method of delivering a prosthetic device into the lumen of a percutaneous transluminal introducer sheath utilizing such a device. The integral front-end loading device facilitates collapsing of the occluder to a narrow configuration which may be flushed directly in the loading device and then delivered into the introducer sheath lumen. Connection of the occluder to the control assembly is reliably and quickly achieved by isolating compatible ball-head ends of the occluder and the locking wire within a narrowly dimensioned bore at the distal tip of the control wire. The present invention also permits the retrieval of an expanded but undeployed occluder, without surgical intervention. Retraction of the expanded occluder against the rigid distal tip of the retrieval sheath ultimately everts the occluder into a configuration which may be withdrawn through the retrieval sheath lumen.

It should be understood that the foregoing description of the invention is intended merely to be illustrative thereof and that other equivalents, embodiments and modifications of the invention may be apparent to those skilled in the art.

## Claims

1. A front-end loading delivery device (12) for transluminal delivery of a collapsible prosthetic occluder (200), comprising:
a main body (21) having a distal end (18), a proximal end and a lumen extending therethrough, a portion (26) of said lumen being tapered inwardly toward a smaller diameter distal portion (28);
a collapsible prosthetic device (200) having a first configuration larger than said distal portion (28) of said lumen and a second configuration that is smaller than said distal portion (28) of said lumen, said collapsible prosthetic device (200) being moveable in said second configuration through said distal portion (28) of said lumen;
**characterized in that**
it further comprises an infusion port (30) extending through said main body (21) and in direct communication with said distal portion (28) of said lumen (26).

2. The front-end loading delivery device recited in claim 1, characterized in that it further comprises an introducer sheath (150, 152), wherein said distal end (28) is constructed and arranged for insertion into a hub (158) at the proximal end of an introducer sheath lumen.

3. The front-end loading delivery device recited in claim 1 wherein said infusion port (30) opens into said lumen distal portion (28) immediately adjacent said inwardly tapered portion (26) of said lumen.

4. The front-end loading delivery device recited in claim 1 wherein said distal portion (28) of said lumen has a uniform diameter.

5. The front-end loading delivery device recited in claim 1 wherein said main body (21) is transparent.

6. The front-end loading delivery device recited in claim 1 further including a valve (32) for sealing said lumen proximally of said smaller diameter distal portion (28).

7. The front-end loading delivery device recited in claim 6 wherein said valve (32) includes a compressible gland (36) having a tapered outer portion (58) constructed and arranged to closely fit in a non-compressed state within a proximal end of said tapered portion (26) of said lumen.

8. The front-end loading delivery device recited in claim 7 wherein said compressible gland (36) further includes a cylindrical portion (56) proximally of said tapered portion (58).

9. The front-end loading delivery device recited in claim 6 wherein said valve (32) includes a cap (34) threadably mounted to said proximal end of said main body (21) and having a lumen (42) extending therethrough and a compressible gland (36) having a lumen (39) in alignment with said cap lumen (42).

10. The front-end loading delivery device recited in claim 9 wherein one of said cap (34) and said gland (36) has an annular groove (50) and the other of said cap and said gland has an annular projection (52) fitted within said groove (50).

11. The front-end loading delivery device recited in claim 9 wherein said cap (34) is internally threaded.

12. The front-end loading delivery device recited in claim 11 wherein said cap (34) includes a locking step projection distally of said internal threads.

13. The front-end loading delivery device recited in claim 9 wherein the perimeter of said cap lumen (42) and said gland lumen (39) in a non-compressed state is smaller than the perimeter of said second configuration of said collapsible prosthetic device (200).

14. The front-end loading delivery device recited in claim 1 wherein said tapered portion (26) of said lumen is conically shaped.

15. The front-end loading delivery device recited in claim 1 wherein said distal end (18) is rigid.

16. A method of delivering a prosthetic device into the lumen of a percutaneous transluminal introducer sheath, comprising:
collapsing the prosthetic device (200) in the lumen of a delivery device (12), the delivery device including the features of the device according to claim 1;
infusing fluid through a port (30) extending into the delivery device and in communication with the delivery device lumen to remove air bubbles from the collapsed prosthetic device (200), and
advancing the prosthetic device (200) from the delivery device (12) into the introducer lumen.

17. The method of delivering a prosthetic device (200) to a percutaneous transluminal introducer sheath (150) recited in claim 16 further comprising the step of sealing a proximal end of the delivery device lumen prior to said infusing step.

18. The method of delivering a prosthetic device (200) into a percutaneous transluminal introducer sheath (150) recited in claim 17 wherein said infusing step includes the step of infusing liquid into the delivery device lumen in a direction perpendicular to the axis of the prosthetic device.

19. The method of delivering a prosthetic device (200) to a percutaneous transluminal introducer sheath (150) recited in claim 16 wherein the prosthetic device (200) includes an umbrella shaped occluder.

## Patentansprüche

1. Frontladeanbringungsvorrichtung (12) zum transluminalen Anbringen einer zusammenklappbaren Verschlußprothese (200), welche folgendes umfaßt:
einen Hauptkörper (21) mit einem distalen Ende (18), einem proximalen Ende und einem sich dadurch erstreckenden Hohlraum, wobei ein Teil (26) dieses Hohlraums sich nach innen aufeinen distalen Teil (28) kleineren Durchmessers verjüngt;
eine zusammenklappbare Prothesenvorrichtung (200) mit einer ersten Konfiguration, die größer als der distale Teil (28) des Hohlraums ist, und einer zweiten Konfiguration, die kleiner als der distale Teil (28) des Hohlraums ist, wobei die zusammenklappbare Prothesenvorrichtung (200) in der zweiten Konfiguration durch den distale Teil (28) des Hohlraums bewegbar ist;
**dadurch gekennzeichnet, daß**
sie weiterhin einen Infusionsport (30) umfaßt, welcher sich durch den Hauptkörper (21) erstreckt und in direkter Verbindung mit dem distalen Teil (28) des Hohlraums (26) steht.

2. Frontladeanbringungsvorrichtung nach Anspruch 1, dadurch gekennzeichnet, daß sie weiterhin eine Einführungsinstrumenthülle (150, 152) umfaßt, wobei das distale Ende (28) zum Einsetzen in ein Ansatzstück (158) an dem proximalen Ende eines Einführungsinstrumenthüllenhohlraums konstruiert und angeordnet ist.

3. Frontladeanbringungsvorrichtung nach Anspruch 1, wobei der Infusionsport (30) sich in den distalen Hohlraumteil (28) unmittelbar neben dem sich nach innen verjüngenden Teil (26) des Hohlraums öffnet.

4. Frontladeanbringungsvorrichtung nach Anspruch 1, wobei der distale Teil (28) des Hohlraums einen gleichmäßigen Durchmesser aufweist.

5. Frontladeanbringungsvorrichtung nach Anspruch 1, wobei der Hauptkörper (21) transparent ist.

6. Frontladeanbringungsvorrichtung nach Anspruch 1, welche weiterhin ein Ventil (32) zum Abdichten des Hohlraums proximal des distalen Teils (28) kleineren Durchmessers beinhaltet.

7. Frontladeanbringungsvorrichtung nach Anspruch 6, wobei das Ventil (32) einen zusammendrückbaren Flansch (36) mit einem sich verjüngenden äußeren Teil (58) beinhaltet, welcher so konstruiert und angeordnet ist, daß er in nicht zusammengedrücktem Zustand in ein proximales Ende des sich verjüngenden Teils (26) des Hohlraums dicht paßt.

8. Frontladeanbringungsvorrichtung nach Anspruch 7, wobei der zusammendrückbare Flansch (36) weiterhin einen zylindrischen Teil (56) proximal des sich verjüngenden Teils (58) beinhaltet.

9. Frontladeanbringungsvorrichtung nach Anspruch 6, wobei das Ventil (32) eine auf dem proximalen Ende des Hauptkörpers (21) schraubbar befestigte und einen sich hierdurch erstreckenden Hohlraum (42) aufweisende Abdeckung (34) sowie einen zusammendrückbaren Flansch (36) mit einem mit dem Abdeckungshohlraum (42) ausgerichteten Hohlraum (39) beinhaltet.

10. Fronladeanbringungsvorrichtung nach Anspruch 9, wobei eines von beiden, nämlich entweder die Abdeckung (34) oder der Flansch (36), eine ringförmige Nut (50) aufweist und das andere von beiden, die Abdeckung bzw. der Flansch, einen in der Nut (50) eingepaßten ringförmigen Vorsprung (52) aufweist.

11. Frontladeanbringungsvorrichtung nach Anspruch 9, wobei die Abdeckung (34) ein Innengewinde aufweist.

12. Frontladeanbringungsvorrichtung nach Anspruch 11, wobei die Abdeckung (34) einen Arretierstufenvorsprung distal der Innengewinde beinhaltet.

13. Frontladeanbringungsvorrichtung nach Anspruch 9, wobei der Umfang des Abdeckungshohlraums (42) und des Flanschhohlraums (39) in nicht zusammengedrücktem Zustand kleiner als der Umfang der zweiten Konfiguration der zusammenklappbaren Prothesenvorrichtung (200) ist.

14. Frontladeanbringungsvorrichtung nach Anspruch 1, wobei der sich verjüngende Teil (26) des Hohlraums konisch ausgebildet ist.

15. Frontladeanbringungsvorrichtung nach Anspruch 1, wobei das distale Ende (18) starr ist.

16. Verfahren zum Anbringen einer Prothesenvorrichtung in dem Hohlraum einer perkutanen transluminalen Einführungsinstrumenthülle welches folgendes umfaßt:
Zusammenklappen der Prothesenvorrichtung (200) in dem Hohlraum einer Anbringungsvorrichtung (12) wobei die Anbringungsvorrichtung die Merkmale der Vorrichtung nach Anspruch 1 beinhaltet;
Infusion von Flüssigkeit durch einen Port (30), der sich in die Anbringungsvorrichtung erstreckt und in Verbindung mit dem Anbringungsvorrichtungshohlraum steht, um Luftbläschen aus der zusammengeklappten Prothesenvorrichtung (200) abzusaugen, und
Vorbewegen der Prothesenvorrichtung (200) von der Anbringungsvorrichtung (12) in die Einführungsinstrumenthülle.

17. Verfahren zum Einbringen einer Prothesenvorrichtung (200) in eine perkutane transluminale Einführungsinstrumenthülle (150) nach Anspruch 16, welches weiterhin den Schritt des Abdichtens eines proximalen Endes des Anbringungsvorrichtungshohlraums vor dem Infusionsschritt umfaßt.

18. Verfahren zum Einbringen einer Prothesenvorrichtung (200) in eine perkutane transluminale Einführungsinstrumenthülle (150) nach Anspruch 17, wobei der Infusionsschritt den Schritt der Infusion von Flüssigkeit in den Anbringungsvorrichtungshohlraum in einer zur Achse der Prothesenvorrichtung senkrechten Richtung beinhaltet.

19. Verfahren zum Einbringen einer Prothesenvorrichtung (200) in eine perkutane transluminale Einführungsinstrumenthülle (150) nach Anspruch 16, wobei die Prothesenvorrichtung (200) einen schirmförmigen Verschluß beinhaltet.

## Revendications

1. Dispositif de transmission à chargement avant (12) pour l'application transluminale d'un élément prothétique d'occlusion pliant (200), comportant :
un corps principal (21) avec une extrémité distale (18), une extrémité proximale et une ouverture s'étendent à travers celui-ci, une partie (26) de ladite ouverture étant diminuée vers l'intérieur vers une partie d'un plus petit diamètre (28) ;
un dispositif prothétique pliant (200) d'une première configuration plus grande que ladite portion distale (28) de ladite ouverture et d'une deuxième configuration qui est plus petite que ladite partie distale (28) de ladite ouverture, ledit dispositif prothétique pliant (200) étant déplaçable dans ladite deuxième configuration à travers ladite partie distale (28) de l'ouverture ;
caractérisé en ce que
il comporte en outre un orifice d'injection (30) s'étendant à travers ledit corps principal (21) et en communication directe avec ladite partie distale (28) de ladite ouverture (26).

2. Dispositif d'application à chargement avant selon la revendication 1, caractérisé en ce qu'il comporte an outre une gaine d'introduction (150, 152), où ladite extrémité distale (28) est construite et agencée pour l'insertion dans un moyeu (158) à l'extrémité proximale d'une ouverture de la gaine d'introduction.

3. Dispositif d'application à chargement avant selon la revendication 1, où ledit orifice d'injection (30) s'ouvre dans ladite partie distale d'ouverture (28) directement adjacente à ladite partie diminuée vers l'intérieur (26) de ladite ouverture.

4. Dispositif d'application à chargement avant selon la revendication 1, où ladite partie distale (28) de ladite ouverture a un diamètre uniforme.

5. Dispositif d'application à chargement avant selon la revendication 1, où ledit corps principal (21) est transparent.

6. Dispositif d'application à chargement avant selon la revendication 1, incluant en outre une vanne (32) pour rendre étanche ladite ouverture proximalement à ladite partie distale de plus petit diamètre (28).

7. Dispositif d'application à chargement avant selon la revendication 6, où ladite vanne (32) comporte un chapeau compressible (36) avec une portion extérieure diminuée (58) construite et agencée pour s'adapter d'une manière serrée dans un état non-comprimé dans l'extrémité proximale de ladite partie diminuée (26) de ladite ouverture.

8. Dispositif d'application à chargement avant selon la revendication 7, où ledit chapeau compressible (36) comporte en outre une partie cylindrique (56) proximalement à ladite partie diminuée (58).

9. Dispositif d'application à chargement avant selon la revendication 6, où ladite vanne (32) comporte un capuchon (34) vissé sur l'extrémité proximale dudit corps principal (21) et comportant une ouverture (42) s'étendant à travers celui-ci ainsi qu'un chapeau compressible (36) avec une ouverture (39) alignée avec ladite ouverture de capuchon (42).

10. Dispositif d'application à chargement avant selon la revendication 9, où l'un parmi ledit capuchon (34) et ledit chapeau (36) présente une rainure annulaire (50) et l'autre dudit capuchon et dudit chapeau a une saillie annulaire (52) adaptée dans ladite rainure (50).

11. Dispositif d'application à chargement avant selon la revendication 9, où ledit capuchon (34) est fileté intérieurement.

12. Dispositif d'application à chargement avant selon la revendication 11, où ledit capuchon (34) comporte une saillie de verrouillage étagée au loin desdits filets intérieurs.

13. Dispositif d'application à chargement avant selon la revendication 9, où le périmètre de ladite ouverture de capuchon (42) et de ladite ouverture de chapeau (39) à l'état non-comprimé, est plus petite que le périmètre de ladite seconde configuration dudit dispositif prothétique pliant (200).

14. Dispositif d'application à chargement avant selon la revendication 1, où ladite partie diminuée (26) de ladite ouverture a une forme conique.

15. Dispositif d'application à chargement avant selon la revendication 1, où ladite extrémité distale (18) est rigide.

16. Procédé pour transmettre un dispositif prothétic dans l'ouverture d'une gaine d'introduction transluminale percutanée, comprenant les étapes consistant à :
plier le dispositif prothétic (200) dans l'ouverture d'un dispositif de transmission (12), le dispositif de transmission incluant les caractéristiques du dispositif selon la revendication 1 ;
injecter du fluide à travers un orifice (30) s'étendant dans le dispositif de transmission et en communication avec l'ouverture du dispositif de transmission pour enlever des bulles d'air du dispositif prothétique plié (200) et,
faire avancer le dispositif prothétique (200) du dispositif de transmission (12) dans l'ouverture d'introduction.

17. Procédé pour transmettre un dispositif prothétique (200) à une gaine d'introduction transluminale percutanée (150) selon la revendication 16, comportant en outre l'étape consistant à rendre étanche une extrémité proximale de l'ouverture du dispositif de transmission avant ladite étape d'injection.

18. Procédé pour transmettre un dispositif prothétique (200) dans une gaine d'introduction transluminale percutanée (150) selon la revendication 17, où ladite étape d'injection comporte l'étape consistant à injecter un liquide dans l'ouverture du dispositif de transmission dans une direction perpendiculaire à l'axe du dispositif prothétique.

19. Procédé pour transmettre un dispositif prothétique (200) à une gaine d'introduction transluminale percutanée (150) selon la revendication 16, où le dispositif prothétique (200) comporte un dispositif d'occlusion en forme de parapluie.
